# EUROPEAN PATENT SPECIFICATION

(11) **EP 2 521 490 B1**
(45) Date of publication and mention of the grant of the patent: **16.09.2015**
(21) Application number: 11702289.7
(22) Date of filing: 04.01.2011
(51) Int. Cl.: A61B 6/00, A61B 6/03, H01J 35/14, H01J 35/30

(54) **X-RAY TUBE WITH A COMBINED X- AND Y- FOCAL SPOT DEFLECTION METHOD**
RÖNTGENRÖHRE MIT KOMBINIERTER X- UND Y-BRENNFLECKABLENKUNG
TUBE À RAYONS X AVEC UN PROCÉDÉ DE DÉVIATION DE FOYER X ET Y COMBINÉ

(30) Priority: 08.01.2010 US 293231 P
(43) Date of publication of application: 14.11.2012
(73) Proprietor: Koninklijke Philips N.V., 5656 AE Eindhoven (NL)
(72) Inventor: KAUTZ, Allan, NL-5656 AE Eindhoven (NL); PERNO, Salvatore, NL-5656 AE Eindhoven (NL); BROOKS, Gary, NL-5656 AE Eindhoven (NL); AWAD, George, NL-5656 AE Eindhoven (NL); BUAN, Jose Angelo, NL-5656 AE Eindhoven (NL)
(74) Representative: van Liempd, Jan
(86) International application number: PCT/IB2011/050013
(87) International publication number: WO 2011/083416

(56) References cited:
- EP-A1- 0 224 786
- WO-A2-2008/044194
- DE-A1- 19 953 613
- US-A- 4 748 650
- US-B1- 6 292 538

## Description

### FIELD OF THE INVENTION

The present invention relates to an X-ray tube, to an X-ray system including such a tube and to a method for a combined deflection of the focal spot in x-direction and in y-direction. Especially, the invention relates to an X-ray tube including means for alternately deflecting the focal spot in x-direction and in y-direction (hence in z-direction). Further, the invention relates to a computer program for controlling an X-ray system including such an X-ray tube.

### BACKGROUND OF THE INVENTION

The concept of deflecting the focal spot to improve resolution of CT systems is well established. Initial applications using single line detectors employed a technique known as x-flying focal spot wherein the focal spot was oscillated in the lateral, or x-direction, about its rest position by approximately one-half pixel. This intentional introduction of parallax effectively doubles the resolution.

Double-x sampling is accomplished by oscillating the focal spot about its static position in a path tangential to the target focal track. The spot is displaced by approximately +/- one half pixel either by using electrostatic grids or by an electromagnet. With double-x sampling, the electron beam/focal spot is deflected laterally to the filament at a prescribed frequency in the x-direction, which is tangential to the target focal track. Samples are taken at each extreme position so that one image contains information obtained at two viewing angles and contains virtually twice the information in a static image. US 6,256,369 discloses the concept of double-x sampling.

The concept of resolution enhancement by focal spot motion has further been refined by the introduction of double-z sampling which has been established in the CT art as a means of increasing resolution and decreasing artifacts. This concept, which is also known as double-z sampling, z-flying focal spot sampling or z-dynamic focal spot sampling involves causing the focal spot to alternate between two z-positions, thus doubling the slice count of a scanner without increasing the number of detector rows. For example, with 32 rows of detectors, this technique effectively measures 64 slices.

Double-z sampling is accomplished essentially by moving the focal spot along a vector primarily directed in the y-direction on the target focal track. Because the focal track is at an angle with regard to the plane of the target (typically 7 or 8 degrees), movement of the focal spot in the y-direction causes it to also move in the z-direction, wherein the distance is proportional to the sine of the target angle. The net effect of double-z sampling is to alternate the focal spot between two z-positions on the detector rows. This effectively acquires two overlapping slices per detector row, for example obtaining 64 slices in 32 rows. It is thus seen that double-z sampling can, in effect, double the slice capability of a CT scanner, turning a 32 slice scanner into a 64 slice scanner.

A recent enhancement to double-z sampling, so-called "quad" sampling involves focal spot deflection in both the x- and z- direction. The technique involves, typically, deflection in the +z direction, followed by deflection in the -x direction, followed by deflection in the -z direction, followed bv deflection in +x direction.

US 6 292 538 B1 discloses an X-ray tube with flying focus that has a magnet system for deflecting and focusing the electron beam.

### SUMMARY OF THE INVENTION

Quad-sampling may be realized by utilizing complex quadrupole magnets that require a fairly high power and also induce interaction effects leading to production of focal spot distortion and thus reduced image quality.

It may therefore be an object of the invention to include capability for both x-flying focal spot and z-flying focal spot with the same CT platform.

It may be a further object to reduce interaction effects between x- and z-flying focal spot generation and a reduction of the required power.

This may be achieved by the subject matter of each independent claim. Further embodiments are described in the respective dependent claims.

According to a first aspect of the present invention an X-ray system may comprise an X-ray tube, a detector for the detection of X-ray beams, and a data processing unit. The X-ray tube maybe adapted to generate X-ray beams and comprises a first deflection device and a second deflection device, wherein both the first and the second deflection device are adapted to deflect a focal spot of the electron beam emitted by a cathode of the X-ray tube diagonally, so that the focal spot has components in both the x- and y- directions. Due to a tapered design of a common X-ray tube anode a deflection in y-direction leads to a deflection in z-direction. In this context, the expression "diagonally" does not necessarily mean that the effective angle of the deflection force caused by the first and second deflection devices relative to the x- axis is 45 degrees.

The first and the second deflection devices may constitute an electromagnetic dipole each. These two dipole magnets may be mounted externally to the X-ray tube or may also be mounted internally and are preferably positioned between the filament and the target so that the electron beam passes between their poles.

In a preferred embodiment the first and second deflection devices are positioned at an equal z-position relative to the X-ray tube, meaning that they are not distanced in z-direction and are situated on the same reference plane. It is understood that this is just one preferred embodiment and the invention is not limited to this embodiment. Hence, the first and second deflection devices may also be distanced in z-direction.

In a preferred embodiment the electromagnetic dipoles are preferably positioned at equal angles (±θ) with respect to the X-ray tube y-axis to produce a diagonal deflection, which means a combined deflection in x- and y-direction. Hence, the magnetic fields have components in both the x- and y-directions.

Commonly used simple dipole electromagnets require only a small amount of magnetic material to produce the required magnetic fields and hence the reluctance can be quite low, facilitating rapidly oscillating the magnetic field and thereby increasing the efficiency of an X-ray system equipped with such an X-ray tube.

According to a second aspect of the present invention the first and second deflection devices are operated independently, meaning that they are never energized both at the same time. Thereby, if they constitute electromagnetic dipoles, their magnetic fields do not interact with each other and thereby no distortion that produces different focal spot shapes in different positions is created.

According to another embodiment, the first deflection device as well as the second deflection device may be operated in both static and dynamic field modes, depending upon input voltage or current. For example, the focal spot may generally be positioned by the use of static fields and may be oscillated about this position by superimposing dynamic fields upon the static field. It is further understood that the design of the X-ray tube may be extended to situations where the first and second deflection devices may not be positioned at equal angles referenced to the X-ray tube y-axis. For example, the first and second deflection devices may be positioned such that focal spot deflections are parallel to the x- and y-directions. Also, the first and second deflection devices may be operated with a variety of polarities.

Furthermore, the cathode of the X-ray tube may comprise more than one filament. The deflection technique may function equally well when more than one filament is present. For example, two filaments may be positioned in such a way that the corresponding electron beams impinge the anode at different focal spots. The first and second deflection devices may deflect both focal spots in an advantageous manner according to the present invention. It may also be advantageous to have filaments with different sizes.

According to a further aspect of the invention, an X-ray system may further comprise a drive control unit controlling a motion of the X-ray tube and the detector relative to an object of interest, and at the same time or independently controlling a motion of the object of interest itself. Such an X-ray system may be for example a computer tomography system, which is also known as a "CT" system.

The X-ray tube according to the invention allows higher powers since the first and second deflection devices produce diagonal deflections. Thereby, the focal spot does not strictly travel in the x-direction and it does not "track" a section of the target focal track. Normally, when the focal spot moves along with the focal track, there is increased heating of that portion of the track. Therefore, the power normally must be reduced to avoid overheating of the target focal track. An X-ray tube according to the present invention can eliminate this disadvantage since the electron beams act on a wider area of the anode.

A further advantage of the invention is that the power supplies driving electromagnetic dipoles as first and second deflection devices could be tied to ground and not have to float at cathode potential.

Further, a method according to the present invention for operating an X-ray system having an X-ray tube as described above and a detector may be beneficial. According to an aspect of the present invention an electron beam in the X-ray tube is deflected diagonally in x- and y-direction, by means of alternately controlling exclusively the first deflection device or the second deflection device according to a predetermined switching sequence. This means, that the first and second deflection devices may be triggered in an alternating manner, such that the first deflection device deflects an electron beam, subsequently the second deflection device deflects an electron beam, further subsequently the first deflection devices deflects an electron beam and so on.

It is preferred that the deflection forces supplied by the first and second deflection devices change their prefixes each time they are triggered. As a result, a predetermined deflection pattern of the focal spot may be achieved.

It will be understood, that the invention may also relate to a computer program element for a data processing unit of an X-ray system as mentioned above. The computer program element may be adapted to control the deflection of the focal spot in x-direction as well as in y-direction, wherein a deflection in z-direction results according to the angular arrangement of the focal track. The computer program may control the first deflection device as well as the separately arranged second deflection device. Preferably, the computer program element is adapted for conducting the method steps described above.

On the other hand, the computer program element may also include instruction for processing the signals received from the detector as a basis for reconstructed two- or three-dimensional images which may be illustrated on a monitor of the system, controlled by the computer program element.

Furthermore, the computer program element may also include instruction for controlling motion of the X-ray tube and the detector relative to an object of interest, and for controlling motion of the object of interest itself relative to the X-ray tube and the detector.

The computer program element may be implemented as a computer-readable instruction code in any suitable programming language, such as, for example, JAVA, C++ and may be stored on a computer-readable medium (removable disk, volatile or non-volatile memory, embedded memory etc.). The instruction code is operable to program a computer or other programmable device to carry out the intended functions and may be loaded into a working memory unit of the computer or other device. The computer program element may be available from a network, such as the WorldWideWeb, from which it may be downloaded.

Also, existing X-ray systems or medical viewing systems may be upgraded with a new software based on the computer program element described above, which, when being executed on a processor, causes the system to carry out the above-mentioned method steps according to the present invention.

It has to be noted that features and side effects of the present invention have been described with reference to different embodiments of the invention. However, a person skilled in the art will gather from the above and the following description that unless other notified in addition to any combination or features belonging to one embodiment also any combinations between features relating to different embodiments or to a manufacturing method is considered to be disclosed with this application.

The aspects defined above and further aspects of the present invention are apparent from the examples of embodiment to be described hereinafter and are explained with reference to the examples of embodiment. The invention will be described in more detail hereinafter for further explanation and better understanding of the present invention with reference to examples of embodiment but to which the invention is not limited. Identical or similar components in different figures are provided with identical reference numerals. The illustrations in the figures are schematic and are not to scale.

### BRIEF DESCRIPTION OF THE DRAWINGS

- Fig. 1: shows an overview of a system according to the present invention.
- Fig. 2: is a schematic illustration of an X-ray tube according to the present invention.
- Fig. 3a: illustrates the positions of the first and second deflection devices.
- Fig. 3b: shows a preferred switching sequence with regard to the cathode and the anode.
- Fig. 4: is an isometric view of an anode including a coordinate system as used in the context of this application.
- Fig. 5: illustrates the relative positions of the first and second deflection devices as well as the anode of an X-ray tube according to the present invention.
- Fig. 6: shows a flow chart with steps of a method for operating an X-ray system according to the present invention.

### DETAILED DESCRIPTION OF EMBODIMENTS

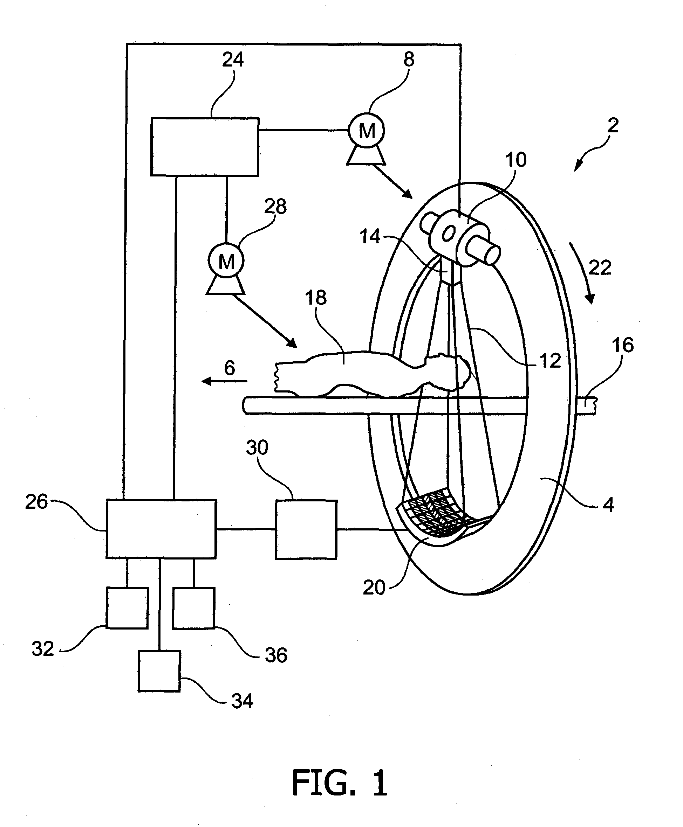

Fig. 1 shows an X-ray system 2 according to the present invention in a schematic overview. The X-ray system 2 may be of a computer tomography type, which is also known as a "CT" scanner in which an X-ray tube according to the invention may be used. The CT scanner 2 comprises a gantry 4, which is rotatably supported around a rotational axis 6 and which is driven by a driving means 8, e.g. an electric motor.

Further, a source of radiation such as an X-ray tube 10 according to the present invention is shown, which may be adapted for emitting a polychromatic radiation. The CT scanner 2 further comprises an aperture system 14, which may be adapted for forming the X-ray radiation being emitted from the X-ray source 10 into a suitable X-ray radiation beam 12. The spectral distribution of the radiation beam emitted from the X-ray tube 10 may further be changed by a filter element (not shown), which may be arranged close to the aperture system 14.

The radiation beam 12, which may comprise a cone shape or a fan shape, is directed to a table 16 such that it penetrates a region of interest, e.g. a head or another body part of a patient 18.

The patient 18 is positioned on the table 16 such that the patient's head is arranged in a central region of the gantry 4, which central region represents the examination region of the CT scanner 2. After penetrating the region of interest the radiation beam 12 impinges onto a radiation detector 20 for acquiring an X-ray image. For suppression of X-ray radiation being scattered by the patient's head and impinging onto the X-ray detector under an oblique angle an anti-scatter-grid may be used which is not depicted in present Fig. 1. The anti-scatter-grid may preferably be positioned directly in front of the detector 20.

The X-ray detector 20 is preferably arranged on the gantry 4 opposite to the X-ray tube 10 according to the present invention. The X-ray detector 20 comprises a plurality of detector elements wherein each detector element is capable of detecting X-ray photons, which have passed through the head of the patient 18.

To improve the scanning process by the above described aspects of the present invention, the initial electron beam emitted from the cathode in the X-ray tube 10 according to the present invention may be deflected by means of a first deflection device and a second deflection device in a combined x-and y-direction. A deflection in y-direction results in a deflection in z-direction due to the tapered margin area of the anode. These elements will be described in more detail below.

During the scanning process of the region of interest, the X-ray tube 10, the aperture system 14 and the X-ray detector 20 are rotated together with the gantry 4, e.g. in a clockwise direction from a view point situated on the left side of Fig. 1, as indicated by arrow 22. For a suitable rotation of the gantry 4, the driving means 8 may be connected to a drive control unit 24, which itself may be connected to a data processing unit 26. The data processing unit 26 may comprise an image reconstruction unit, which may be realized by means of hardware and/or by means of software constituting a part of a computer program element according to the present invention. The reconstruction unit may be adapted to reconstruct a three-dimensional image based on a plurality of two-dimensional images obtained under different observation angles. These may additionally be adjusted by means of suitable electron beam deflections using first and second deflection devices in the X-ray tube 10.

It is preferred that the data processing unit 26 also serves as a control unit for communicating with the drive control unit 24 in order to coordinate the movement of the gantry 4 with the movement of the table 16. A linear displacement of the table 16 is carried out by a driving means 28, which may also be connected to the drive control unit 24.

During the operation of the X-ray system 2 according to the present invention the gantry 4 may conduct a rotational motion wherein at the same time the table 16 may be shifted parallel to the rotational axis 6 such that a helical scan of the region of interest may be performed. It should be noted that it may be also possible to perform a circular scan in that no displacement in a direction parallel to the rotational axis 6 is conducted and merely a rotational motion of the gantry 4 is provided. Thereby, two-dimensional X-ray images ("slices") of the head may be acquired with a high accuracy. A three-dimensional representation of the patient's head may be obtained through the reconstruction unit in the data processing unit 26 by sequentially moving the table 16 in discrete steps parallel to the rotational axis 6 after for example at least one-half gantry rotation has been performed for each discrete table position.

The X-ray detector 20 may be connected to the data processing unit 26 through a pre-amplifier 30 for retrieving an amplified analogue signal from the X-ray detector 20. Based on a plurality of different X-ray projection data sets, which have been acquired at different projection angles, a three-dimensional representation of the patient's head is then reconstructed.

For observing a three dimensional reconstruction of the region of interest a display 32 is provided, which is connected to the data processing unit 26. Additionally, arbitrary slices of perspective views of the three-dimensional representation may also be printed out using a printer 34, which may also be connected to the data processing unit 26. Further, the data processing unit 26 may also be connected to an image archiving and communication system 36 for storing acquired images and three-dimensional reconstructions respectively.

It should be noted that the display 32, the printer 34 and/or the other devices supplied in the X-ray system 2 according to the present invention may be positioned in the vicinity of the X-ray system 2 according to the present invention so that observation of the scanning process and/or acquired image data is easily possible. Alternatively, these components may be located in a remote position from the X-ray system 2 according to the present invention, such as in a adjacent room, in a completely other location within an institution or hospital, where the X-ray system 2 according to the present invention is located, or in an entirely different location linked to the X-ray system 2 according to the present invention by use of one or more configurable networks, such as the Internet, Virtual Private Networks (VPN) or the such.

Furthermore, the data processing unit 26 is preferably connected with the X-ray tube 10 according to the present invention. By way of this connection, the data processing unit 26 is enabled to control the focal spot deflection in x- and y-direction by directly and alternately triggering the first and second deflection devices, respectively.

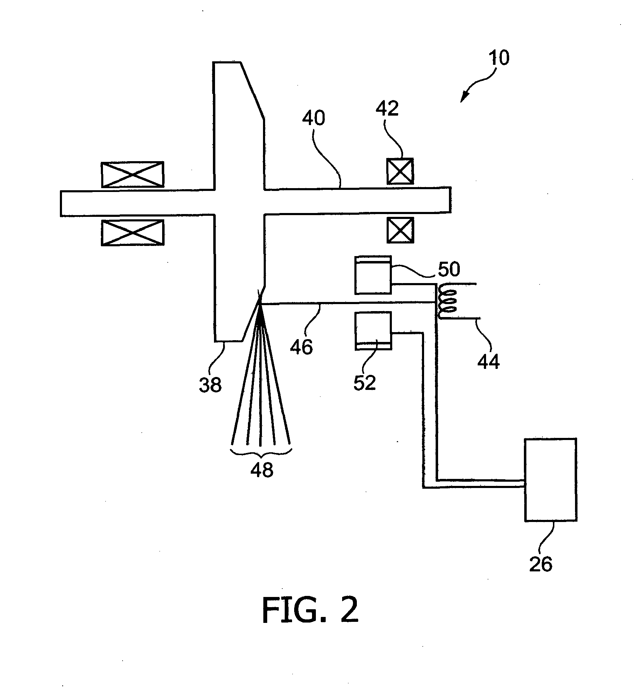

Fig. 2 shows an X-ray tube 10 according to the present invention, which is adapted to generate X-ray beams originated from different X-ray focal spots. The X-ray tube 10 comprises an anode 38 having a shaft 40, which shaft 40 is rotatably supported such that it may be rotated around the z-axis. A rotational driving means 42 is connected to the shaft 40 via a mechanical and/or a magnetic coupling and allows for a rotational movement of the anode 38 at a fairly large rotational speed.

The X-ray tube 10 according to the present invention further comprises an electron source or filament or cathode 44, which is arranged laterally with respect to the z-axis. According to the embodiment described herein, the electron source 44 may be realized as a hot cathode generating an electron beam 46 during operation, which electron beam 46 impinges onto a tapered surface of the anode 38. Thereby, a focal spot is defined. Since it is tapered, the anode surface where the electron beam 46 is impinging onto is orientated oblique with respect to the z-axis such that from the focal spot an X-ray beam 48 projects radially outwards from the z-axis.

For a precise control of the focal spot the X-ray tube 10 according to the present invention comprises a first deflection device 50 and a second deflection device 52, wherein the first deflection device and the second deflection device are exemplarily realized as two electromagnetic coils each or as an electromagnetic dipole, each adapted for deflecting the electron beam 46 in the x-direction and in the y-direction (and hence in the z-direction) at the same time. This can be achieved by a diagonal mounting position of the first and second deflection devices around the neck between the cathode cup with the electron source 44 and the anode 38. It is to be noted that although stating deflection devices 50 and 52 in singular form, a deflection device 50 and 52 may also refer to two electromagnetic coils each. It is understood that a deflection device 50 or 52 may be built up by two electromagnetic coils each. To simplify matters in the following only the expression "deflection device" will be used.

The first and second electron deflection devices 50 and 52 are preferably connected with the control unit 26, which provides necessary electric signals to the first and second electron deflection devices 50 and 52.

The first and second electron deflection devices 50 and 52 may independently deflect the electron beam 40 as they may independently be triggered through the control unit 26. According to the present invention this eliminates interaction effects between the first and second deflection devices 50 and 52. This means, that exclusively the first deflection device 50 or the second deflection device 52 are activated.

In case the first and second deflection devices are realized as electromagnetic dipoles it is noted that these dipoles may also be constituted by a U-shaped magnetic element each with only one electromagnetic coil located around the bottom of the U-shape between the legs of the U-shape.

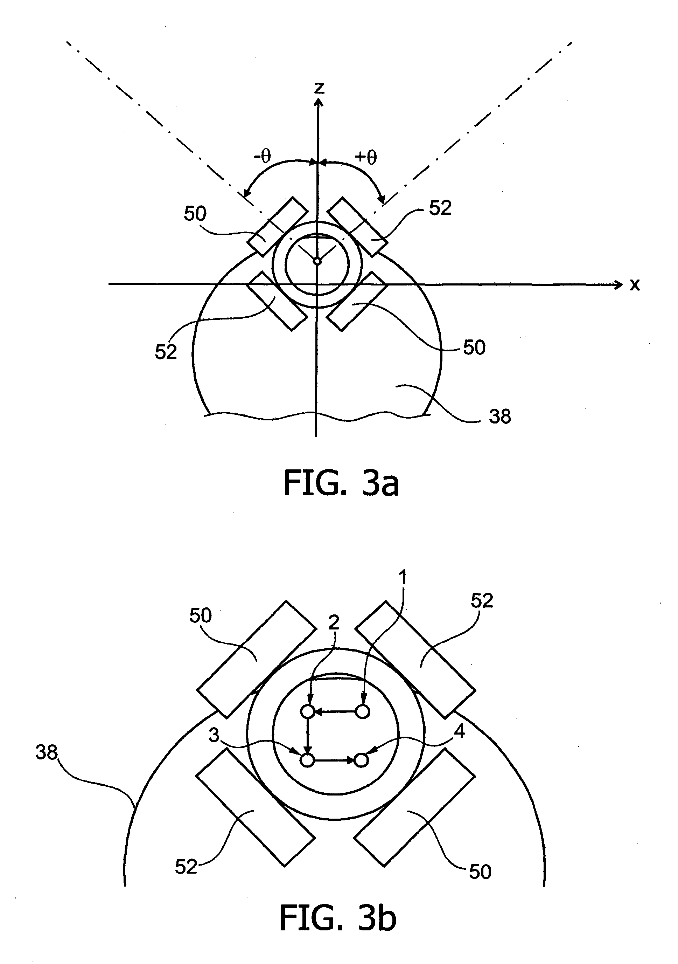

Fig. 3a illustrates the positions of the first and second deflection devices 50 and 52. The first deflection device 50 constitutes an electromagnetic dipole with two separate electromagnetic coils. The angle between a connection line within the two electromagnetic coils of the first deflection device 50 and the y-axis is -θ. The angle between a connection line within the two electromagnetic coils of the second deflection device 52 and the x-axis is +θ. The first and second deflection devices 50 and 52 are preferably positioned between the filament/cathode 44 and the target/anode 38 so that the electron beam 46 passes between their poles.

It can easily be gathered from Fig. 3a that on activating one of the first or second deflection devices an electron beam emitted from the cathode 44 is deflected both in x-and y-direction.

It is noted that the coordinate system of an X-ray system 2 according to the present invention is arranged conforming the conventional directions of x, y and z. This coordinate system is referenced to a patient 18 as can be seen in Fig. 1. The x-direction is lateral to the patient, the y-direction is vertical to the patient, and the z-direction is along the length of the patient.

According to a central aspect of the present invention the first and second deflection devices 50 and 52 are activated independently from each other and in an alternating manner. For continuously achieving a higher image quality a continuous deflection or switching sequence is necessary. Referring to Figure 3b, one preferred switching sequence would be 1.-2.-3.-4., wherein this sequence corresponds to [dipole/second deflection device 52 (+on), dipole/first deflection device 50 off], [dipole/first deflection device 50 (+on), dipole/second deflection device 52 off], [dipole/second deflection device 52 (-on), dipole/first deflection device 50 off], [dipole/first deflection device 50 (-on), dipole/second deflection device 52 off]. Thereby, no spot on the focal track is "tracked" twice which reduces the risk of overheating. The present invention is not limited to this switching sequence as other ways are possible but it is to be understood that only one coil is energized at a time and the switching sequence produced a focal spot deflection in an advantageous manner.

Preferably, this switching sequence may be realized by providing a suitably conditioned pulse width modulation ("PWM") signal to the first and second deflection devices 50 and 52.

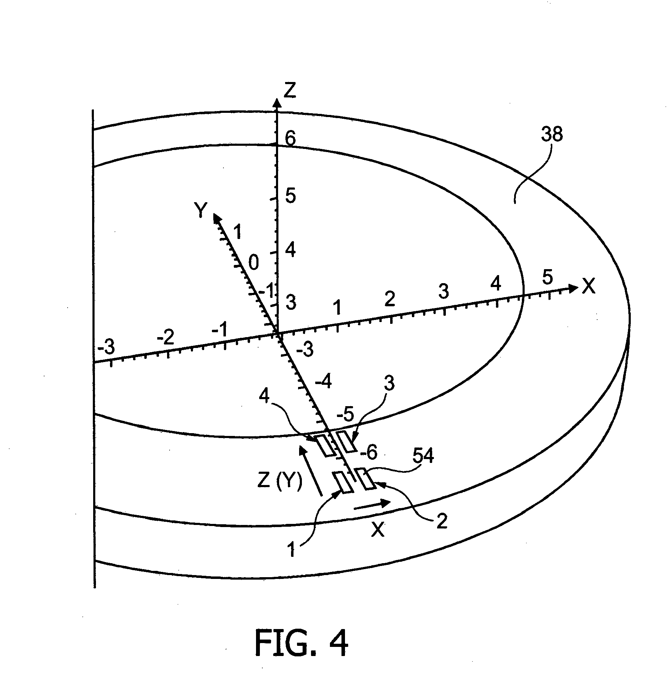

In Fig. 4 the four deflected focal spot positions 54 according to the above switching sequence on the target area of the anode 38 are shown. The deflection in the z-direction is conducted by deflecting the electron beam 46 in the y-direction. This causes the beam to walk up the tapered margin/track of the anode 38, thus causing the focal spot to move also into the z-direction.

The x-deflection results in obtaining information of an object at two different viewing angles and thus improving the resolution, since the image contains virtually twice the information than a static image. The z-deflection provides the ability to acquire images at a double slice count.

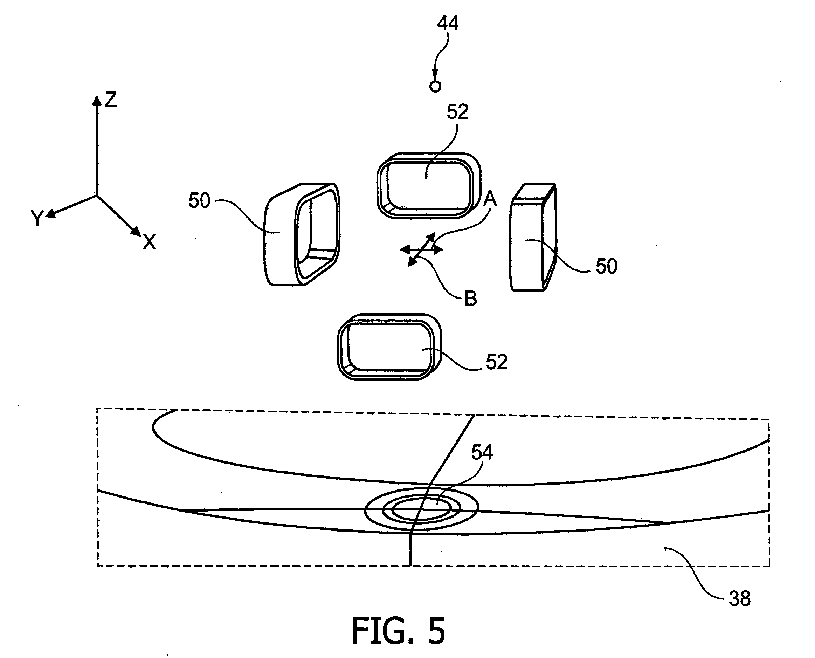

Fig. 5 shows a detailed view of the arrangement of the first deflection device 50, the second deflection device 52 and a section of the anode 38. It can be seen that the first deflection device 50 and the second deflection device 52 are not mounted along the x- and the y-axes. The electron emitting cathode 44 is shown that emits an electron beam towards the anode 38. Coming from the cathode 44, the electron will pass through the intermediate spaces of the first and second deflection devices 50 and 52. On activation of the first or the second deflection device 50 or 52 the electron beam will be deflected by means of the respective magnetic field. The arrow "A" in Fig. 5 stands for activation of the first deflection device 50 with two electromagnetic coils as an electromagnetic dipole. The arrow "B" stands for the second deflection device, built up by a second electromagnetic dipole.

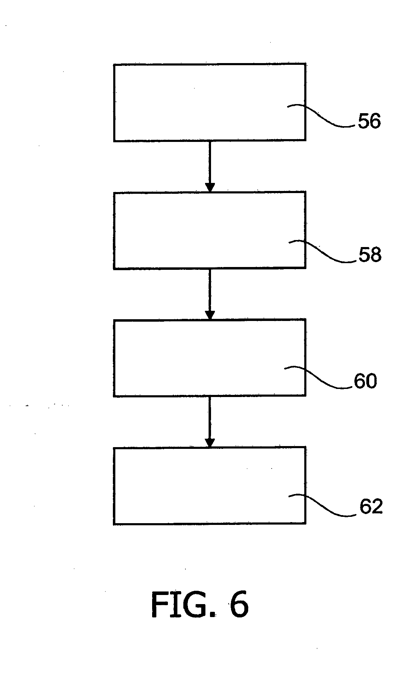

Finally, Fig. 6 shows a flow chart with exemplary steps for a method of operating an X-ray system 2 according to the present invention. These steps are preferably implemented as instructions within a computer program element according to the present invention. All mentioned steps may be understood as major, superordinate steps that may also comprise subordinate steps that are not explicitly mentioned hereinafter.

First of all, the X-ray tube 10 according to the present invention and the detector 20 are positioned relative to the object of interest, by means of controlling 56 the driving means 8 and 28.

Subsequently, an electron beam emitted by the cathode 44 in the X-ray tube 10 according to the present invention, is deflected in x- and y-direction, by means of exclusively controlling 58 the first deflection device 50 or the second deflection device 52 according to a predetermined switching sequence.

Further, in step 60 the deflected electron beam leaves the X-ray tube 10 according to the present invention and penetrates the object of interest, impinging the detector elements of the detector 20.

Finally, images are generated 62 by the data processing unit 26 on the basis of the signals received from the detector elements 20.

In a next pass of the method steps according to the present invention the deflection another deflection direction of the electron beam 46 is achieved by further iterating through the predetermined switching sequence.

While the invention has been illustrated and described in detail in the drawings and foregoing description, such illustration and description are to be considered illustrative or exemplary and not restrictive. The invention is not limited to the disclosed embodiments.

Other variations to the disclosed embodiments can be understood and effected by those skilled in the art in practicing the claimed invention, from a study of the drawings, the disclosure and the appended claims. In the claims the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage.

### LIST OF REFERENCE SIGNS:

- 2: X-ray system
- 4: gantry
- 6: rotational axis
- 8: driving means
- 10: X-ray tube
- 12: radiation beam
- 14: aperture system
- 16: table
- 18: patient
- 20: detector
- 22: direction of rotation
- 24: drive control unit
- 26: data processing unit
- 28: driving means
- 30: pre-amplifier
- 32: display
- 34: printer
- 36: communication system
- 38: anode
- 40: shaft
- 42: driving means
- 44: electron source/cathode
- 46: electron beam
- 48: X-ray beam
- 50: first deflection device / electromagnetic coils
- 52: second deflection device / electromagnetic coils
- 54: focal spot position
- 56: controlling driving means
- 58: controlling first deflection device or second deflection device
- 60: detector signal generation
- 62: image generation

## Claims

1. An X-ray system (2) comprising an X-ray tube (10) adapted to generate X-ray beams, a detector (20) for the detection of X-ray beams and a data processing unit, the X-ray tube comprising
an rotating anode (38), wherein a focal spot moving along a focal track defines an x-direction, tangential to the focal track, and an y-direction, perpendicular to the x-direction, the x- and y-direction being perpendicular to the rotation axis of the anode
a first deflection device (50) for focal spot deflection in a combined x- and y-direction, and
a second deflection device (52) for focal spot deflection in a combined x- and y-direction,
**characterised in that**
the first and second deflection devices are separate devices and are arranged diagonally relative to the y-direction and the x-direction of the X-ray tube (10), and
wherein the X-ray system (2) is adapted for exclusively and alternately deflecting an electron beam in the X-ray tube (10) diagonally in x- and y-direction, by means of alternately controlling exclusively the first deflection device (50) or the second deflection device (52) according to a predetermined switching sequence.

2. The X-ray system of claim 1, wherein the first deflection device (50) and second deflection device (52) include an electromagnetic dipole each.

3. The X-ray system of claim 1, wherein the first deflection device (50) and second deflection device (52) are mounted between a cathode (44) and an anode (38) of the X-ray tube (10) at the same position in z-direction.

4. The X-ray system of claim 1, wherein the X-ray tube (10) is adapted for exclusively activating the first deflection device (50) or second deflection device (52)

5. The X-ray system of claim 1, wherein the first deflection device (50) is operated to generate a field out of the group consisting of a static field, a dynamic field and a static field superimposed by a dynamic field.

6. The X-ray system of claim 1, wherein the second deflection device (52) is operated to generate a field out of the group consisting of a static field, a dynamic field and a static field superimposed by a dynamic field.

7. The X-ray system of claim 1, comprising a cathode (44) having one or more filaments.

8. The X-ray system of claim 1, further comprising a drive control unit (24) controlling a motion of the X-ray tube (10) and detector (20) relative to an object of interest (18), and controlling a motion of the object of interest.

9. A method for operating the X-ray system (2) according to claim 1, the method comprising the step:
- exclusively and alternately deflecting an electron beam in the X-ray tube (10) diagonally in x- and y-direction, by means of alternately controlling (58) exclusively a first deflection device (50) or a second deflection device (52) according to a predetermined switching sequence.

10. The method according to claim 9, wherein the deflection of the focal spot according to the switching sequence is conducted in a way that no spot of the focal track is tracked more than once during one switching sequence.

11. The method according to claim 9, further comprising the step:
- positioning the X-ray tube (10) and the detector (20) relative to the object of interest.

12. A computer program element including instructions which, when executed on the X-ray system (2) according to claim 1, causing the first deflection device (50) or the second deflection device (52) of the X-ray tube (10) of the X-ray system (2) to exclusively and alternately deflect the focal spot of the electron beam diagonally in the x-direction and y-direction at the same time according to a predetermined switching sequence.

13. The computer program element of claim 12, further including instructions for processing signals received from the detector (20), to generate images of an object of interest (18), and for illustrating the images on a display (32).

14. The computer program element of claim 12, further including instructions for controlling motion of the X-ray tube (10) and detector (20) relative to an object of interest (18), and for controlling motion of the object of interest relative to the X-ray tube and detector.

## Patentansprüche

1. Röntgensystem (2) mit einer Röntgenröhre (10), die dafür ausgelegt ist, Röntgenstrahlen zu erzeugen, einem Detektor (20) zum Erkennen der Röntgenstrahlen und einer Datenverarbeitungseinheit, wobei die Röntgenröhre Folgendes umfasst:
eine Drehanode (38), wobei ein sich entlang einer Brennfleckbahn bewegender Brennfleck eine x-Richtung tangential zu der Brennfleckbahn und eine y-Richtung senkrecht zu der x-Richtung definiert, wobei die x- und die y-Richtung senkrecht zu der Drehachse der Anode verlaufen;
eine erste Ablenkungsvorrichtung (50) zum Ablenken des Brennflecks in eine kombinierte x- und y-Richtung, und
eine zweite Ablenkungsvorrichtung (52) zum Ablenken des Brennflecks in eine kombinierte x- und y-Richtung,
**dadurch gekennzeichnet, dass** die erste und die zweite Ablenkungsvorrichtung getrennte Vorrichtungen sind und diagonal in Bezug auf die y-Richtung und die x-Richtung der Röntgenröhre (10) angeordnet sind, und
wobei das Röntgensystem (2) dafür ausgelegt ist, ausschließlich und abwechselnd ein Elektronenstrahlenbündel in der Röntgenröhre (10) diagonal in x- und y-Richtung abzulenken, indem abwechselnd ausschließlich die erste Ablenkungsvorrichtung (50) oder die zweite Ablenkungsvorrichtung (52) entsprechend einer vorgegebenen Schaltsequenz angesteuert wird.

2. Röntgensystem nach Anspruch 1, wobei die erste Ablenkungsvorrichtung (50) und die zweite Ablenkungsvorrichtung (52) jeweils einen elektromagnetischen Dipol umfassen.

3. Röntgensystem nach Anspruch 1, wobei die erste Ablenkungsvorrichtung (50) und die zweite Ablenkungsvorrichtung (52) zwischen einer Kathode (44) und einer Anode (38) der Röntgenröhre (10) an der gleichen Position in z-Richtung montiert sind.

4. Röntgensystem nach Anspruch 1, wobei die Röntgenröhre (10) dafür ausgelegt ist, ausschließlich die erste Ablenkungsvorrichtung (50) oder die zweite Ablenkungsvorrichtung (52) zu aktivieren.

5. Röntgensystem nach Anspruch 1, wobei die erste Ablenkungsvorrichtung (50) betrieben wird, um ein Feld ausgewählt aus der Gruppe bestehend aus einem statischen Feld, einem dynamischem Feld und einem statischem Feld, das durch ein dynamisches Feld überlagert wird, zu erzeugen.

6. Röntgensystem nach Anspruch 1, wobei die zweite Ablenkungsvorrichtung (52) betrieben wird, um ein Feld ausgewählt aus der Gruppe bestehend aus einem statischen Feld, einem dynamischem Feld und einem statischem Feld, das durch ein dynamisches Feld überlagert wird, zu erzeugen.

7. Röntgensystem nach Anspruch 1, das eine Kathode (44) mit einem oder mehreren Heizdrähten umfasst.

8. Röntgensystem nach Anspruch 1, weiterhin mit einer Antriebssteuereinheit (24), die eine Bewegung der Röntgenröhre (10) und des Detektors (20) relativ zu einem interessierenden Objekt (18) steuert und die eine Bewegung des interessierenden Objekts steuert.

9. Verfahren zum Betrieb des Röntgensystems (2) nach Anspruch 1, wobei das Verfahren den folgenden Schritt umfasst:
- ausschließlich und abwechselnd Ablenken eines Elektronenstrahlenbündels in der Röntgenröhre (10) diagonal in x- und y-Richtung, indem abwechselnd ausschließlich die erste Ablenkungsvorrichtung (50) oder die zweite Ablenkungsvorrichtung (52) entsprechend einer vorgegebenen Schaltsequenz angesteuert (58) wird.

10. Verfahren nach Anspruch 9, wobei die Ablenkung des Brennflecks entsprechend der Schaltsequenz auf derartige Weise erfolgt, dass während einer einzelnen Schaltsequenz kein Fleck der Brennfleckbahn mehr als ein einzelnes Mal verfolgt wird.

11. Verfahren nach Anspruch 9, das weiterhin den folgenden Schritt umfasst:
- Positionieren der Röntgenröhre (10) und des Detektors (20) in Bezug auf das interessierende Objekt.

12. Computerprogrammelement mit Anweisungen, die, wenn sie auf dem Röntgensystem (2) nach Anspruch 1 ausgeführt werden, die erste Ablenkungsvorrichtung (50) oder die zweite Ablenkungsvorrichtung (52) der Röntgenröhre (10) des Röntgensystems (2) veranlassen, ausschließlich und abwechselnd den Brennfleck des Elektronenstrahlenbündels diagonal in der x-Richtung und der y-Richtung gleichzeitig entsprechend einer vorgegebenen Schaltsequenz abzulenken.

13. Computerprogrammelement nach Anspruch 12, das weiterhin Anweisungen zum Verarbeiten von Signalen, die vom Detektor (20) empfangen wurden, umfasst, um Bilder eines interessierenden Objekts (18) zu erzeugen und um die Bilder auf einer Anzeige (32) darzustellen.

14. Computerprogrammelement nach Anspruch 12, das weiterhin Anweisungen zum Steuern der Bewegung der Röntgenröhre (10) und des Detektors (20) in Bezug auf ein interessierendes Objekt (18) und zum Steuern der Bewegung des interessierenden Objekts in Bezug auf die Röntgenröhre und den Detektor umfasst.

## Revendications

1. Système de radiographie (2) comprenant un tube à rayons X (10) adapté pour générer des faisceaux de rayons X, un détecteur (20) pour la détection de faisceaux de rayons X et une unité de traitement de données, le tube à rayons X comprenant :
une anode rotative (38), dans lequel un foyer se déplaçant le long d'une piste focale définit une direction x, tangentielle à la piste focale, et une direction y, perpendiculaire à la direction x, les directions x et y étant perpendiculaire à l'axe de rotation de l'anode,
un premier dispositif de déflexion (50) pour la déflexion de foyer dans des directions x et y combinées, et
un second dispositif de déflexion (52) pour la déflexion de foyer dans des directions x et y combinées,
dans lequel les premier et second dispositifs de déflexion sont des dispositifs séparés et sont agencés diagonalement par rapport à la direction y et à la direction x du tube à rayons X (10), et
dans lequel le système de radiographie (2) est adapté pour défléchir exclusivement et en alternance un faisceau d'électrons dans le tube à rayons X (10) diagonalement dans les directions x et y, au moyen de la commande alternée exclusivement du premier dispositif de déflexion (50) ou du second dispositif de déflexion (52) selon une séquence de permutation prédéterminée.

2. Système de radiographie selon la revendication 1, dans lequel le premier dispositif de déflexion (50) et le second dispositif de déflexion (52) comprennent un dipôle électromagnétique chacun.

3. Système de radiographie selon la revendication 1, dans lequel le premier dispositif de déflexion (50) et le second dispositif de déflexion (52) sont montés entre une cathode (44) et une anode (38) du tube à rayons X (10) à la même position dans la direction z.

4. Système de radiographie selon la revendication 1, dans lequel le tube à rayons X (10) est adapté pour activer exclusivement le premier dispositif de déflexion (50) ou le second dispositif de déflexion (52).

5. Système de radiographie selon la revendication 1, dans lequel le premier dispositif de déflexion (50) est mis en fonctionnement pour générer un champ parmi le groupe constitué d'un champ statique, d'un champ dynamique et d'un champ statique sur lequel est superposé un champ dynamique.

6. Système de radiographie selon la revendication 1, dans lequel le second dispositif de déflexion (52) est mis en fonctionnement pour générer un champ parmi le groupe constitué d'un champ statique, d'un champ dynamique et d'un champ statique sur lequel est superposé un champ dynamique.

7. Système de radiographie selon la revendication 1, comprenant une cathode (44) possédant un ou plusieurs filaments.

8. Système de radiographie selon la revendication 1, comprenant en outre une unité de commande d'entraînement (24) commandant un mouvement du tube à rayons X (10) et du détecteur (20) par rapport à un objet d'intérêt (18), et commandant un mouvement de l'objet d'intérêt.

9. Procédé pour faire fonctionner un système de radiographie (2) selon la revendication 1, le procédé comprenant l'étape :
- la déflexion exclusive et alternée d'un faisceau d'électrons dans le tube à rayons X (10) diagonalement dans les directions x et y, au moyen de la commande alternée (58) exclusivement d'un premier dispositif de déflexion (50) ou d'un second dispositif de déflexion (52) selon une séquence de permutation prédéterminée.

10. Procédé selon la revendication 9, dans lequel la déflexion du foyer selon la séquence de permutation est réalisée de manière telle qu'aucun point de la piste focale ne soit suivi plus d'une fois durant une séquence de permutation.

11. Procédé selon la revendication 9, comprenant en outre l'étape :
- le positionnement du tube à rayons X (10) et du détecteur (20) par rapport à l'objet d'intérêt.

12. Élément de programme d'ordinateur comprenant des instructions qui, lorsqu'elles sont exécutées le système de radiographie (2) selon la revendication 1, font en sorte que le premier dispositif de déflexion (50) ou le second dispositif de déflexion (52) du tube à rayons X (10) du système de radiographie (2) défléchisse exclusivement ou en alternance le foyer du faisceau d'électrons diagonalement dans la direction x et la direction y en même temps selon une séquence de permutation prédéterminée.

13. Élément de programme d'ordinateur selon la revendication 12, comprenant en outre des instructions pour traiter des signaux reçus à partir du détecteur (20), pour générer des images d'un objet d'intérêt (18), et pour illustrer les images sur un affichage (32).

14. Élément de programme d'ordinateur selon la revendication 12, comprenant en outre des instructions pour commander le mouvement du tube à rayons X (10) et du détecteur (20) par rapport à un objet d'intérêt (18), et pour commander le mouvement de l'objet d'intérêt par rapport au tube à rayons X et au détecteur.
